# FASCICULE DE BREVET EUROPEEN

(11) **EP 0 745 374 B1**
(45) Date de publication et mention de la délivrance du brevet: **24.09.1997**
(21) Numéro de dépôt: 96401110.0
(22) Date de dépôt: 22.05.1996
(51) Int. Cl.: A61K 7/15, A61K 7/00

(54) **Gel de rasage à moussage différé contenant un monoester d'acide en C4-C10 et d'alcool en C16-C18**
Verzögert schäumendes Rasiergel, das einen Monoester einer C4-C10 Säure und eines C16-C18 Alkohols enthält
Delayed foaming shaving gel containing a monoester of an C4-C10 acid and a C16-C18 alcohol

(30) Priorité: 02.06.1995 FR 9506608
(43) Date de publication de la demande: 04.12.1996
(73) Titulaire: L'OREAL, 75008 Paris (FR)
(72) Inventeur: Devaine, André, 95190 Goussainville (FR); Caudet, Alain, 92190 Meudon (FR)
(74) Mandataire: Andral, Christophe André Louis

(56) Documents cités:
- EP-A- 0 259 843
- EP-A- 0 339 634
- WO-A-95/05147
- DE-A- 1 961 146

## Description

La présente invention concerne un nouveau gel aqueux de rasage à moussage différé du type comprenant, un savon hydrosoluble, un agent de moussage volatil dit 〈〈 à effet différé 〉〉, et éventuellement un polymère gélifiant hydrosoluble, et qui est caractérisé par le fait qu'il comprend en outre un ester spécifique qui est un monoester d'acide aliphatique en C₄-C₁₀ et d'alcool aliphatique en C₁₆-C₁₈.

Les gels à moussage différé (ou à effet de mousse différé) sont déjà connus dans le domaine cosmétique, notamment dans leurs applications comme gels de rasage, et ils ont été décrits dans de nombreux brevets tels que, par exemple, US 3 541 581, US 4 405 489, et FR 2 595 943.

Ces gels à effet de mousse différé sont des compositions généralement conditionnées sous pression dans un dispositif aérosol qui délivre, sous l'effet d'un propulseur, des gels non moussants dans des conditions statiques, mais qui, sous l'action mécanique due à l'étalement du produit sur la peau, engendrent spontanément et presque instantanément une mousse sur cette dernière.

Les gels aqueux de rasage à moussage différé doivent réunir de nombreuses caractéristiques qui rendent leur mise au point particulièrement difficile.

Tout d'abord, le gel délivré dans la main doit présenter une rhéologie appropriée (rigidité, consistance). En outre, à l'étalement sur la peau du visage, ce gel doit pouvoir développer très rapidement une mousse, elle-même susceptible de prendre du volume sans toutefois produire des petits amas de mousse rigide. Enfin, la mousse engendrée par le gel doit être dotée de bonnes qualités cosmétiques, telles que l'apport de douceur, et l'absence d'effet collant et/ou d'effet filant sur la peau.

Malheureusement, les compositions de gel de rasage à moussage différé de l'art antérieur ne remplissent généralement pas l'ensemble de ces conditions.

Pour faciliter la mise en oeuvre et augmenter la stabilité de tels gels, on a déjà proposé d'y introduire des alcanolamides d'acides gras en C₁₂-C₁₈, ou des alcools gras éthoxylés, ou bien encore du dipelargonate de propylène glycol. Cependant, là encore, les gels obtenus n'ont pas réuni toutes les propriétés ci-dessus énoncées et ne se sont donc pas encore révélés totalement satisfaisants.

La présente invention vise à proposer des gels de rasage à moussage différé présentant des propriétés améliorées.

Ainsi, après de nombreuses recherches menées sur la question, la Demanderesse a maintenant découvert, de façon totalement inattendue et surprenante, qu'en ajoutant un monoester d' acide aliphatique en C₄-C₁₀ et d'alcool aliphatique en C₁₆-C₁₈, à la composition d'un gel de rasage classique comprenant en milieu aqueux, (a) un savon hydrosoluble, (b) un agent de moussage volatil dit 〈〈 à effet différé 〉〉, et (c) éventuellement un polymère gélifiant hydrosoluble, on pouvait obtenir un gel présentant des performances et/ou des qualités améliorées, et ceci par le fait qu'il présente toutes les caractéristiques recherchées décrites précédemment.

Cette découverte est à la base de la présente invention.

La présente invention a ainsi pour objet un nouveau gel aqueux de rasage à moussage différé, du type comprenant un savon hydrosoluble, un agent de moussage volatil dit 〈〈 à effet différé 〉〉 et éventuellement un polymère gélifiant hydrosoluble, et qui est caractérisé par le fait qu'il comprend en outre au moins un monoester d' acide aliphatique en C₄-C₁₀ et d'alcool aliphatique en C₁₆-C₁₈.

Le ou les monoesters d'acides aliphatiques en C₄-C₁₀ et d'alcools aliphatiques en C₁₆-C₁₈ qui peuvent être utilisés selon l'invention sont de préférence choisis parmi les monoesters d'acides aliphatiques saturés linéaires ou ramifiés en C₄-C₁₀ et d'alcools aliphatiques saturés linéaires en C₁₆-C₁₈.

Des monoesters de ce type plus particulièrement préférés selon la présente invention sont l'ester de l'acide heptanoique et de l'alcool stéarylique (ou heptanoate de stéaryle), l'ester de l'acide octanoique et de l'alcool stéarylique (ou octanoate de stéaryle), ou encore des mélanges heptanoate / octanoate de stéaryle, en particulier le mélange heptanoate (67%) / octanoate (33%) de stéaryle vendu par exemple par la société CRODA sous la dénomination commerciale CRODAMOL W, ou par la société STEARINERIES DUBOIS sous la dénomination commerciale DUB SOLIDE.

Les autres constituants rentrant dans la composition des gels de rasage à effet de mousse différé conformes à l'invention sont des produits déjà connus en soi dans l'application considérée (voir à cet égard EP-A-259 843, US 3 541 581 et FR 2 595 943).

Le savon hydrosoluble est de préférence un sel hydrosoluble d'acide gras. De tels savons sont bien connus de l'art antérieur, existent dans le commerce ou peuvent être préparés selon les méthodes conventionnelles, par exemple par réaction d'une base, telle que la triéthanolamine, directement sur un acide gras, tel qu'un acide gras saturé ou non saturé en C₁₀ à C₂₂, ou des mélanges de ces acides. Des savons préférés selon l'invention comprennent les stéarates, myristates et palmitates hydrosolubles, tels que les savons aminés solubles des acides stéarique ou palmitique commerciaux. Les sels de triéthanolamine de ces acides sont plus particulièrement préférés. Par ailleurs, il est bien connu que le produit commercial vendu sous le nom d'acide stéarique peut être un mélange d'acides stéarique et palmitique. Le terme 〈〈 stéarates〉〉 désigne les savons d'acide stéarique du commerce, mais peut également désigner les savons d'acide stéarique pur.

Un savon hydrosoluble particulièrement préféré selon l'invention est le palmitate de triéthanolamine.
Lorsqu'un polymère gélifiant hydrosoluble est utilisé selon l'invention, il peut être choisi parmi les hydroxyalkylcelluloses hydrosolubles ou les gommes naturelles telles que la gomme de xanthane. Les hydroxyalkylcelluloses sont fabriquées à partir d'une alkyl cellulose et d'un oxyde d'alkylène tel que l'oxyde d'éthylène ou l'oxyde de propylène. Des produits de ce type sont vendus sous les marques 〈〈KLUCEL〉〉 et 〈〈NATROSOL〉〉 dans une gamme de viscosités variées.
Un polymère hydrosoluble plus particulièrement préféré selon la présente invention est une hydroxypropylcellulose vendue sous la marque 〈〈KLUCEL H〉〉 ou 〈〈KLUCEL MF〉〉 par la société AQUALON, une hydroxyéthylcellulose vendue sous la marque 〈〈NATROSOL 250 HHR〉〉 par la société AQUALON, ou un mélange de ces deux celluloses.

L'un des avantages attachés à la présente invention est que l'on peut réduire, voire supprimer, la quantité d'épaississant cellulosique dont les proportions trop élevées sont à l'origine de l'inconvénient du collant sur la peau.

L'agent de moussage différé est liquide ou liquéfiable, et volatil à la température de la peau. Il comprend les hydrocarbures aliphatiques saturés comportant de 4 à 6 atomes de carbone, tels que les butanes, n-butane ou isobutane, les pentanes, tels que le n-pentane ou l'isopentane, ou encore l'hexane. Des mélanges de ces hydrocarbures peuvent être utilisés pour obtenir la pression de vapeur désirée.
Un agent de moussage différé particulièrement bien adapté pour régler la pression du gel en vue d'obtenir les qualités de mousse requises pour la présente invention est le mélange isopentane (75% en poids) / isobutane (25% en poids).

L'eau, présente dans la composition du gel selon l'invention, participe aux qualités recherchées de la mousse, sert à humidifier la peau et à assurer un rasage convenable.

Dans les gels de rasage à moussage différé selon la présente invention, le monoester d' acide aliphatique en C₄-C₁₀ et d'alcool aliphatique en C₁₆-C₁₈ est généralement présent dans des concentrations pondérales (ramenées à l'ensemble de la composition) comprises entre environ 0,5 et 5%, de préférence entre environ 1,5 et 3%, le savon hydrosoluble est généralement présent dans des concentrations pondérales comprises entre environ 5 et 20%, de préférence entre environ 10 et 15%, le polymère gélifiant hydrosoluble peut être présent dans des concentrations pondérales comprises entre environ 0 et 3%, de préférence entre environ 0 et 1% selon la viscosité du produit employé, l'agent de moussage différé est généralement présent dans des concentrations pondérales comprises entre environ 0,5 et 10%, de préférence entre environ 2 et 5%, l'eau est généralement présente dans des concentrations pondérales comprises entre environ 60 et 90%, par rapport au poids total des compositions.

Le gel de rasage à moussage différé selon l'invention peut encore contenir d'autres ingrédients ou actifs bien connus dans le domaine des produits de rasage, tels que par exemple, des hydratants, pour n'en citer que quelques uns, la glycérine et le sorbitol, des agents apaisants tels que l'allantoine ou l'α-bisabolol, des lubrifiants tels que des silicones ou des polydécènes, des émollients comme le polyéthylèneglycol, le polypropylèneglycol ou le monostéarate de glycérol, des agents tensio-actifs, des vitamines, des solvants, des colorants, parfums et conservateurs.

Bien entendu, l'homme de l'art veillera à choisir le ou les éventuels composés complémentaires mentionnés ci-avant, de manière telle que les propriétés avantageuses attachées intrinséquement au gel selon l'invention ne soient pas ou substantiellement pas, altérées par la ou les adjonctions envisagées.

Les gels selon l'invention peuvent être conditionnés sous forme de tubes ou d'aérosols, selon des techniques bien connues de l'homme de l'art et en particulier décrites dans US 3 541 581.
Les dispositifs aérosol peuvent être à simple ou à double enveloppe.

Dans les dispositifs aérosol à double enveloppe, le système propulseur est séparé du gel conforme à l'invention. En effet, on introduit le gel conforme à l'invention dans la partie centrale de l'aérosol à double enveloppe, on introduit le propulseur dans l'enveloppe extérieure qui est séparée de la partie centrale par un diaphragme qui est une membrane plastique compressible.
Dans de tels dispositifs à double enveloppe, le système propulseur peut alors être constitué d'un gaz condensable tel qu'un hydrocarbure comme le propane, le butane, l'isobutane, l'isopentane, les hydrocarbures halogénés, ou le diméthyléther, ou encore les mélanges de ces composés. Il peut également être constitué d'un mélange de ces agents propulseurs condensables avec des gaz non condensables tels que l'oxyde nitreux ou l'azote.

Dans les dispositifs à simple enveloppe, le système propulseur est uniquement constitué de gaz non condensables, insolubles dans le gel conforme à l'invention, tel que l'azote, l'argon, le néon, le krypton, le xénon, l'hélium, le radon, l'oxyde nitreux ou le gaz carbonique.
De tels dispositifs sont équipés d'un tube plongeant à la partie supérieure de laquelle on introduit ledit système propulseur. Dans ce cas, le système propulseur non condensable et insoluble dans le gel conforme à l'invention agit comme un piston et expulse le gel par le tube plongeant.

Dans les aérosols, le gel à l'état pressurisé (ou 〈〈 jus 〉〉) représente avantageusement de 90 à 98% du poids de l'ensemble de la composition, et le système propulseur de 2 à 10% de ce poids.

Les gels selon l'invention sont utilisables pour le rasage des poils et/ou des cheveux présents sur la peau humaine ou animale.

Des exemples concrets, mais nullement limitatifs, illustrant l'invention vont maintenant être donnés.

### EXEMPLE 1 :

On prépare un gel de rasage à moussage différé de la façon suivante ; Dans la partie centrale d'un dispositif aérosol à double enveloppe, on introduit (i) 97 grammes de la composition suivante :

| | |
|---|---|
| Acide palmitique | 7,5 g |
| Acide stéarique | 2,5 g |
| Sorbitol | 2,0 g |
| Propylèneglycol | 2 g |
| Hydroxyéthylcellulose (Natrosol 250 HHR de la société AQUALON) | 0,25 g |
| Tensio-actif: Lauryléthersulfate de triéthanolamine | 2,5 g |
| Polydiméthylsiloxane | 1 g |
| Heptanoate (67%) / octanoate (33%) de stéaryle (Dub Solide de la société STEARINERIES DUBOIS) | 3 g |
| Triéthanolamine | 7,5 g |
| Eau déminéralisée qsp | 100 g |

et (ii) 3 grammes de l'agent de moussage différé constitué d'un mélange isopentane (75% poids) / isobutane (25% poids).

On ferme la partie centrale. On procède ensuite à la pressurisation du mélange obtenu ci-dessus ou 〈〈 jus 〉〉, en introduisant à la partie inférieure de la double enveloppe, et par l'opercule du fond, 4 à 10 grammes d'un propulseur constitué d'un mélange isobutane (55%poids), butane (22%), propane (23%) vendu sous la dénomination Aérogaz 3,2N par la société ELF AQUITAINE.

L'aérosol serti est alors prêt à être utilisé. Lorsqu'il est actionné, il délivre dans la main un gel d'une rhéologie telle qu'elle lui confère une rigidité lui permettant de tenir dans la main et de s'étaler avec facilité de façon homogène sur la peau du visage sans engendrer de petits amas de mousse. Sous l'action mécanique de l'étalement sur la peau, le gel développe rapidement une mousse, douce à l'application, onctueuse et régulière, qui ne colle pas à la peau et ne file pas. Après rasage, la peau est douce et lisse.

### EXEMPLE 2 :

On prépare un gel de rasage à moussage différé de la façon suivante : Dans la partie centrale d'un dispositif aérosol à double enveloppe, on introduit (i) 97 grammes de la composition suivante :

| | |
|---|---|
| Acide palmitique | 12, g |
| Acide myristique | 3, g |
| Glycérine | 1, g |
| Monostéarate de glycérol | 0,1 g |
| Tensio-actif : Octyl phénol oxyéthyléné par 5 moles d'oxyde d'éthylène | 2, g |
| Polydécène (Silkflo S 362 NF de la société ALBEMARLE) | 1, g |
| Heptanoate (67%) / octanoate (33%) de stéaryle (Dub Solide de la société STEARINERIES DUBOIS) | 3, g |
| Triéthanolamine | 9, g |
| Eau déminéralisée qsp | 100, g |

et (ii) 3 grammes d'un agent de moussage différé constitué d'un mélange isopentane (75%poids) / isobutane (25% poids)

On ferme la partie centrale. On procède ensuite à la pressurisation du jus de la même manière que suivant l'exemple 1.

Le gel de rasage ainsi préparé est utilisé de la même façon qu'à l'exemple précédent et présente les mêmes propriétés.

### EXEMPLE 3:

On prépare un gel de rasage à moussage différé de la façon suivante : Dans la partie centrale d'un dispositif aérosol à double enveloppe, on introduit (i) 97 grammes de la composition suivante :

| | |
|---|---|
| Acide palmitique | 11, g |
| Triéthanolamine | 8,5 g |
| Glycérine | 4, g |
| Hydroxypropylcellulose (Klucel MF de la société AQUALON) | 0,2 g |
| Polyéthylèneglycol (Polyox WSR 205 de la société AMERCHOL) | 0,3 g |
| Heptanoate (67%) / octanoate (33%) de stéaryle (Dub Solide de la société STEARINERIES DUBOIS) | 3, g |
| Polydiméthylsiloxane | 1, g |
| α-Bisabolol | 0,1 g |
| Eau deminéralisée qsp | 100, g |

et (ii) 3 grammes d'un agent de moussage différé constitué d'un mélange isopentane (75%poids) / isobutane (25% poids)

On ferme la partie centrale.
On procède ensuite à la pressurisation du jus de la même manière que suivant l'exemple 1, mais en utilisant 4 à 6 grammes d'un propulseur constitué d'isobutane vendu sous la dénomination Aeron Isobutane par la société KLOCKNER.

Le gel de rasage ainsi préparé est utilisé de la même façon qu'aux exemples précédents et présente les mêmes propriétés.

## Revendications

1. Gel aqueux de rasage à moussage différé, du type comprenant un savon hydrosoluble, un agent de moussage différé volatil et éventuellement un polymère gélifiant hydrosoluble, caractérisé par le fait qu'il comprend en outre au moins un monoester d'acide aliphatique en C₄-C₁₀ et d'alcool aliphatique en C₁₆-C₁₈.

2. Gel selon la revendication 1, caractérisé par le fait que ledit monoester est choisi parmi les heptanoates de stéaryle, les octanoates de stéaryle ou leurs mélanges.

3. Gel selon la revendication 2, caractérisé par le fait que ledit monoester est un mélange heptanoate / octanoate de stéaryle.

4. Gel selon l'une quelconque des revendications précédentes, caractérisé par le fait que ledit savon est choisi parmi les sels de l'acide stéarique ou myristique ou palmitique, ou encore d'un mélange de ces acides.

5. Gel selon la revendication 4, caractérisé par le fait que ledit savon est choisi parmi les sels de triéthanolamine des acides stéarique, myristique ou palmitique, ou encore d'un mélange de ces sels.

6. Gel selon l'une quelconque des revendications précédentes, caractérisé par le fait que ledit agent de moussage différé volatil est choisi dans le groupe constitué des butanes, des pentanes, des hexanes et de leurs mélanges.

7. Gel selon la revendication 6, caractérisé par le fait que ledit agent de moussage différé volatil est un mélange d'isopentane et d'isobutane.

8. Gel selon l'une quelconque des revendications précédentes, caractérisé par le fait que ledit polymère gélifiant est choisi parmi les hydroxypropylcelluloses, les hydroxyéthylcelluloses, ou un mélange de ces composés.

9. Gel selon l'une quelconque des revendications précédentes, caractérisé par le fait que ledit ester est présent dans des concentrations pondérales comprises entre 0,5 et 5% et de préférence entre 1,5 et 3% en poids par rapport au poids total de la composition.

10. Gel selon l'une quelconque des revendications précédentes, caractérisé par le fait que ledit savon est présent dans des concentrations comprises entre 5 et 20% et de préférence entre 10 et 15% en poids par rapport au poids total de la composition.

11. Gel selon l'une quelconque des revendications précédentes, caractérisé par le fait que ledit agent de moussage différé est présent dans des concentrations comprises entre 0,5 et 10% et de préférence entre 2 et 5% en poids par rapport au poids total de la composition.

12. Gel selon l'une quelconque des revendications précédentes, caractérisé par le fait que ledit polymère gélifiant est présent dans des concentrations comprises entre 0 et 3% et de préférence entre 0 et 1% en poids par rapport au poids total de la composition.

13. Gel selon l'une quelconque des revendications précédentes, caractérisé par le fait que l'eau est présente à raison de 60 à 90% en poids par rapport au poids total de la composition.

14. Gel selon l'une quelconque des revendications précédentes, caractérisé par le fait qu'il se présente dans un état pressurisé sous la forme d'un aérosol.

15. Utilisation d'un gel tel que défini à l'une quelconque des revendications précédentes pour le rasage des poils ou des cheveux.

## Claims

1. Aqueous shaving gel with delayed foaming, of the type including a water-soluble soap, a volatile delayed-foaming agent and optionally a water-soluble gelling polymer, characterized in that it additionally includes at least one monoester of C₄-C₁₀ aliphatic acid and of C₁₆-C₁₈ aliphatic alcohol.

2. Gel according to Claim 1, characterized in that the said monoester is chosen from stearyl heptanoates, stearyl octanoates and their mixtures.

3. Gel according to Claim 2, characterized in that the said monoester is a stearyl heptanoate/octanoate mixture.

4. Gel according to any one of the preceding claims, characterized in that the said soap is chosen from the salts of stearic or myristic or palmitic acid or else of a mixture of these acids.

5. Gel according to Claim 4, characterized in that the said soap is chosen from the triethanolamine salts of stearic, myristic or palmitic acids or else of a mixture of these salts.

6. Gel according to any one of the preceding claims, characterized in that the said volatile delayed-foaming agent is chosen from the group consisting of butanes, pentanes, hexanes and of their mixtures.

7. Gel according to Claim 6, characterized in that the said volatile delayed-foaming agent is a mixture of isopentane and isobutane.

8. Gel according to any one of the preceding claims, characterized in that the said gelling polymer is chosen from hydroxypropyl celluloses, hydroxyethyl celluloses or a mixture of these compounds.

9. Gel according to any one of the preceding claims, characterized in that the said ester is present in weight concentrations of between 0.5 and 5 % and preferably between 1.5 and 3 % by weight relative to the total weight of the composition.

10. Gel according to any one of the preceding claims, characterized in that the said soap is present in concentrations of between 5 and 20 % and preferably between 10 and 15 % by weight relative to the total weight of the composition.

11. Gel according to any one of the preceding claims, characterized in that the said delayed-foaming agent is present in concentrations of between 0.5 and 10 % and preferably between 2 and 5 % by weight relative to the total weight of the composition.

12. Gel according to any one of the preceding claims, characterized in that the said gelling polymer is present in concentrations of between 0 and 3 % and preferably between 0 and 1 % by weight relative to the total weight of the composition.

13. Gel according to any one of the preceding claims, characterized in that water is present in a proportion of 60 and 90 % by weight relative to the total weight of the composition.

14. Gel according to any one of the preceding claims, characterized in that it is in a pressurized state in the form of an aerosol.

15. Use of a gel as defined in any one of the preceding claims for shaving hair.

## Patentansprüche

1. Wässeriges nachschäumendes Rasiergel, das eine wasserlösliche Seife, ein flüchtiges nachschäumendes Mittel und gegebenenfalls ein wasserlösliches gelbildendes Polymer enthält,
**dadurch gekennzeichnet, daß**
es ferner mindestens einen Monoester einer aliphatischen C₄₋₁₀-Säure und eines aliphatischen C₁₆₋₁₈-Alkohols enthält.

2. Gel nach Anspruch 1, dadurch gekennzeichnet, daß der Monoester unter den Stearylheptanoaten, Stearyloctanoaten und deren Gemischen ausgewählt ist.

3. Gel nach Anspruch 2, dadurch gekennzeichnet, daß der Monoester ein Gemisch von Stearylheptanoat und Stearylocatnoat ist.

4. Gel nach einem der vorhergehenden Ansprüche, dadurch gekennzeichnet, daß die Seife unter den Salzen von Stearinsäure oder Myristinsäure oder Palmitinsäure oder auch Gemischen dieser Säuren ausgewählt ist.

5. Gel nach Anspruch 4, dadurch gekennzeichnet, daß die Seife unter den Triethanolaminsalzen von Stearinsäure, Myristinsäure oder Palmitinsäure oder Gemischen dieser Salze ausgewählt ist.

6. Gel nach einem der vorhergehenden Ansprüche, dadurch gekennzeichnet, daß das flüchtige nachschäumende Mittel unter den Butanen, Pentanen, Hexanen und deren Gemischen ausgewählt ist.

7. Gel nach Anspruch 6, dadurch gekennzeichnet, daß das flüchtige nachschäumende Mittel ein Gemisch von Isopentan und Isobutan ist.

8. Gel nach einem der vorhergenden Ansprüche, dadurch gekennzeichnet, daß das gelbildende Polymer unter den Hydroxypropylcellulosen, Hydroxyethylcellulosen oder Gemischen dieser Verbindungen ausgewählt ist.

9. Gel nach einem der vorhergehenden Ansprüche, dadurch gekennzeichnet, daß der Ester in Konzentrationen im Bereich von 0,5 bis 5 Gew.-% und vorzugsweise von 1,5 bis 3 Gew.-%, bezogen auf das Gesamtgewicht der Zusammensetzung, vorliegt.

10. Gel nach einem der vorhergehenden Ansprüche, dadurch gekennzeichnet, daß die Seife in Konzentrationen im Bereich von 5 bis 20 und vorzugsweise von 10 bis 15 Gew.%, bezogen auf das Gesamtgewicht der Zusammensetzung, vorliegt.

11. Gel nach einem der vorhergehenden Ansprüche, dadurch gekennzeichnet, daß das nachschäumende Mittel in Konzentrationen im Bereich von 0,5 bis 10 Gew.-% und vorzugsweise von 2 bis 5 Gew.-%, bezogen auf das Gesamtgewicht der Zusammensetzung, vorliegt.

12. Gel nach einem der vorhergehenden Ansprüche, dadurch gekennzeichnet, daß das gelbildende Polymer in Konzentrationen von 0 bis 3 Gew.-% und vorzugsweise von 0 bis 1 Gew.-%, bezogen auf das Gesamtgewicht der Zusammensetzung, vorliegt.

13. Gel nach einem der vorhergehenden Ansprüche, dadurch gekennzeichnet, daß das Wasser in Mengenanteilen von 60 bis 90 Gew.-%, bezogen auf das Gesamtgewicht der Zusammensetzung, vorliegt.

14. Gel nach einem der vorhergehenden Ansprüche, dadurch gekennzeichnet, daß es unter Druck in Form eines Aerosols vorliegt.

15. Verwendung eines Gels nach einem der vorhergehenden Ansprüche zum Rasieren von Körperhaar oder Kopfhaar.
